# EUROPEAN PATENT APPLICATION

(11) **EP 1 925 326 A2**
(43) Date of publication of application: **28.05.2008**
(21) Application number: 07020680.0
(22) Date of filing: 23.10.2007
(51) Int. Cl.: A61L 29/04, A61L 29/16

(54) **Bacteriostatically operating tube construction for food or medical use**

(30) Priority: 27.11.2006 IT MI20062275
(71) Applicant: Officine Lodigiane S.r.l., 26867 Somaglia (LO) (IT)
(72) Inventor: Santandrea, Marco, 26867 Somaglia Lodi (IT)
(74) Representative: Cicogna, Franco

(57) **Abstract**

The present invention relates to an extruded plastic material tube, including, in its inside, during the extruding process therefor, one or more spiral or coil elements, made of a silver material, with a bacteriostatic function. The tube is particularly suitable to be used in devices for processing and delivering beverages, in particular water, or for medical applications.

## Description

### BACKGROUND OF THE INVENTION

The present invention relates to a bacteriostatically operating tube construction for food or medical use.

The tube construction of the present invention can be used in devices for processing and delivering beverages, in particular water, or in like devices, in the food or medical field, and in all applications requiring to properly disinfect a fluid being conveyed through a tube.

As is known, silver, Ag, CAS 7740-22-4, is used from a lot of time as a biocide material.

Practical tests have shown that silver or salts thereof provides an efficient disinfecting action for water to be consumed by human beings.

Actually, silver (together with other metals) is provided with oligodynamic properties, that is, in other words, it is suitable to provide a disinfecting effect, even in a very low amount, on water to be consumed by human beings.

Moreover, silver is not toxic for the human organism or body.

Furthermore, the processing of drinking water by silver does not generate objectable odors, colors or taste in water being processed, and does not generate any disinfection byproducts.

### SUMMARY OF THE INVENTION

The aim of the present invention is to provide such a tube construction having a bacteriostatic function, specifically for food or medical use, and adapted to exploit in an optimum manner the biocide or disinfecting characteristics of silver.

Within the scope of the above mentioned aim, a main object of the invention is to provide such a tube construction having a very low load loss, with respect to its application in hydraulic systems.

Yet another object of the present invention is to provide such a tube construction which is very competitive and economically advantageous with respect to prior like biocide or disinfecting tube systems.

Yet another object of the present invention is to provide such a tube construction adapted to solve a lot of different problems affecting the prior art, both from a quality standpoint of the product being delivered, both from a making standpoint, thereby said tube construction can be easily and safely used in a lot of devices and apparatus for processing water for human consume.

According to one aspect of the present invention, the above mentioned aim and objects, as well as yet other objects, which will become more apparent hereinafter, are achieved by a bacteriostatically operating tube construction, specifically designed for food or medical use, characterized in that said tube construction comprises an extruded plastic material tubular body containing in its inside one or more silver elements.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:
Figure 1 is a partially cross-sectioned perspective view showing a portion of a tube construction adapted to bacteriostatically operate, according to the present invention;
Figure 2 is a further longitudinal cross-sectioned perspective view showing an extruding process diagram for extruding the tube construction according to the present invention;
Figure 3 is a further partially broken-away perspective view of a portion of a bacteriostatically operating tube construction, according to a further aspect of the present invention, and comprising a silver wire arranged in a linear manner inside the tube;
Figure 4 is a cross-sectional view showing a bacteriostatically operating tube construction, according to a further aspect of the present invention, comprising a polygonal cross-section tubular element made of silver;
Figure 5 is a further cross-sectional perspective view showing the tube construction shown in the preceding figure 4;
Figure 6 is yet another cross-sectional view of a bacteriostatically operating tube construction, according to a further aspect of the present invention, comprising a silver element including a silver bar or rod;
Figure 7 is a further cross-sectional perspective view of the tube construction shown in the preceding figure;
Figure 8 is yet another cross-sectional view of a bacteriostatically operating tube construction, according to a further aspect of the present invention, comprising a rectangular cross-section tubular element, made of silver;
Figure 9 is yet another cross-sectional perspective view of the tube construction shown in the preceding figure;
Figure 10 is yet another cross-sectional view showing a bacteriostatically operating tube construction, according to a further aspect of the present invention, comprising a silver element including a plurality of silver pieces arranged in a linear manner along an inner wall of the tube construction;
Figure 11 is yet another cross-sectional perspective view of the tube construction shown in the preceding figure;
Figure 12 is yet another cross-sectional view of a bacteriostatically operating tube construction, according to a further aspect of the present invention, comprising a silver cell element;
Figure 13 is yet another cross-sectional perspective view of the tube construction shown in the preceding figure;
Figure 14 is yet another cross-sectional view of a bacteriostatically operating tube construction, according to a further aspect of the present invention, comprising a triangular cross-sectional tubular element made of a silver materials:
Figure 15 is yet another cross-sectional perspective view of the tube construction shown in figure 14;
Figure 16 is a further cross-sectional view of a bacteriostatically operating tube construction, according to a further aspect of the present invention, comprising a silver element including three longitudinal angled portions;
   and
figure 17 is yet another perspective view, as cross-sectioned, of the tube construction of the preceding figure.

### DESCRIPTION OF THE PREFERRED EMBODIMENTS

With reference to the number references of the above mentioned figures, the bacteriostatically operating tube construction according to the present invention, which has been generally indicated by the reference number 1, comprises an extruded plastic material tubular body, generally indicated by the reference number 2, including, in its inside, one or more elements 3 made of a silver material.

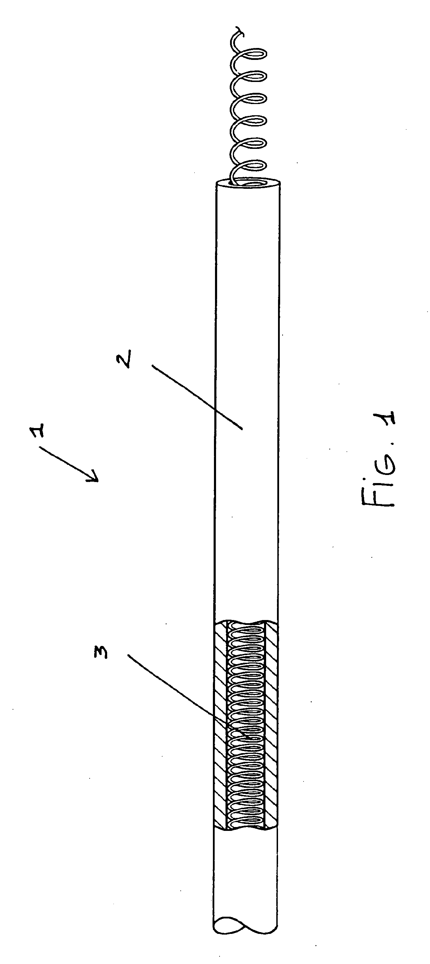

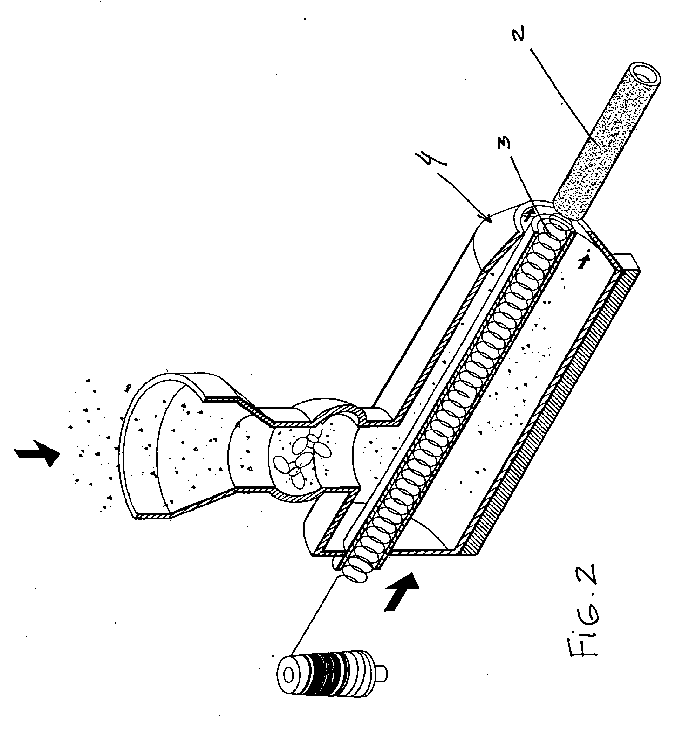

Figures 1 and 2 show a preferred embodiment of the invention, in which the silver element comprises a coiled silver wire.

More specifically, the tubular body is made by a plastic material extruding process, i.e. a process for extruding polyurethane, polyethylene, rilsan®, polyamide, hytrel, the extruded pipes or tubes being covered, and having a tube inside, in which, during the extruding process, are formed and introduced one or more coil elements made of a 999/1000 silver wire.

In particular, the silver wire used for performing application tests was of a type having a silver rate of 999/1000, and a circular cross-section having a diameter of 0.5 mm.

A set of tests have been carried out by using different cross-section and diameter wires, the obtained results being optimum in all cases.

More specifically, the present invention provides to use a silver coil or spiral element, without any shape, cross-section or diameter limitations.

Actually, during control tests, have been examined applications in which the inventive tube has been used for conveying water of average salt composition, polluted by bacteric strains, to control possible proliferations of colonies inside the tube portion being tested.

Moreover, during the car.ried out tests, the coil element pitch values, temperature conditions, flow-rates and pressures have been changed, to verify, for the highest pressures, the load losses due to the presence of the spiral coil or coil element inside the tube.

Moreover, a further test has been carried out, by using colored water, to control or verify a passing through dynamics of the fluid inside the tube, and therefrom a formation of vortexes has been found as occurring.

The results of the performed tests were always positive results.

The bacterial loads did not grow, but they decreased and, in some cases, it has not been possible to detect them.

The coil pitch, that is the distance between two coils or spirals, which has been found to be an optimum result for all tubes having a diameter less than 12 mm, corresponded to 20 mm.

It should be apparent that such a pitch value can be changed, depending on the coil number and the coil cross-sections.

With respect to the found load loss, that is a pressure decreasing due to inner frictions caused by the provision of the metal coil element, it has been found that the mentioned load loss was distributed, owing to a continuous and constant provision of the coil inside the tube, with an increase of the loss value from 15% to 18% with respect to the same tube without any inner coil elements.

It is to be pointed out that, for a particular application in an apparatus for processing and delivering beverages, the mentioned load loss due to the provision of the silver coil in the subject tube construction is very small, with respect to a proper hydraulic designing of the subject system.

With respect to the test for controlling the duration and efficiency of the silver material coil wire, in its bacteriostatic use in the tube, the data have been theoretically established, by considering the average values related to the releasing through water of silver ions under normal conditions.

This control has been carried out by using a polyethylene tube, like that generally used in apparatus for delivering beverages, and having an outer diameter of 8 mm, and an inner diameter of 6 mm.

A circular cross-section silver wire has been herein used, having a diameter of 0.5 mm, a pitch (the distance between two coil turns) of 20 mm, and, considering the daily water volume as processed per day by a beverage for water delivery apparatus, it is possible to provide a time efficiency of the apparatus exceeding 10 years.

With respect to the industrial making of the subject tube extruded construction, as indicated by the reference number 2, with an inner silver coil or spiral element 3, a conventional or prior extruding head, having a generally solid central core, for forming the tube construction passage, has been so modified as to allow a silver wire to be passed therethrough.

In particular, in the extruding head, generally indicated by the reference 4 in figure 2, the mentioned passage or tube lumen has been provided by forming a scroll path, to aid the forming of the target coil element and its even engaging inside the tube.

A suitably timed feeding or advancing system, allows to provide a constant silver wire feeding depending on the extruding speed of the plastic material tube.

Due to the combined action as caused by a conventional plastic tube shrinking during its cooling, and because of a slight embedding of the silver wire in the tube plastic material being solidified, and due to the very small increase of the silver coil element diameter, it is possible to properly lock the spiral element 3 inside the tube construction 2.

It has been found that the invention fully achieves the intended aim and objects.

In fact, the extruded plastic material tube construction, including an inner silver coil or spiral element, according to the present invention, constitute a novel approach allowing to greatly reduce the bacteric proliferating risk in hydraulic circuits, in particular as the tube is used in water processing devices, designed for processing water to be used in human consume.

Actually, the recognized efficiency of silver as disinfecting antibacteric material, even if it is present in a very small amount, its absence of toxicity for the human body, jointly with its properties of a full absence of objectable odors, colors or taste, and moreover due to the fact that it does not generate disinfection byproducts, it is an ideal material for disinfecting water for human consume.

With respect to the cost related to its use, because of a minimum amount used, and due to its comparatively long duration with a comparatively high efficiency, it is a competitive and economically preferable material, with respect to other biocide materials.

Accordingly, the invention solves a plurality of problems affecting the prior art water disinfection methods, in the terms of the quality of the obtained and delivered product, the cost of the processes, and the safety and managing apparatus problems, related to the devices or apparatus for processing water for human consume.

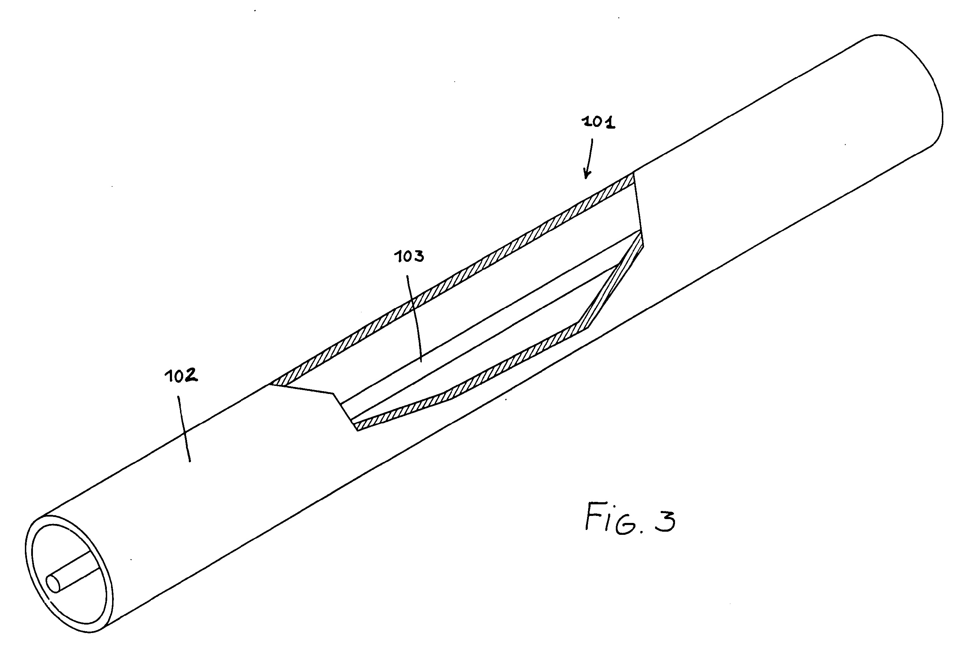

Figures 3 to 17 show, by way of an example, some other possible embodiments of the invention, in which the silver element is differently made.

In figure 3, for example, is shown a portion of a bacteriostatically operating tube construction, according to the present invention, generally indicated by the reference number 101, comprising an extruded plastic material tubular body, generally indicated by the reference number 102, in which a silver element including a silver wire 103, linearly arranged in said tube 102, is provided.

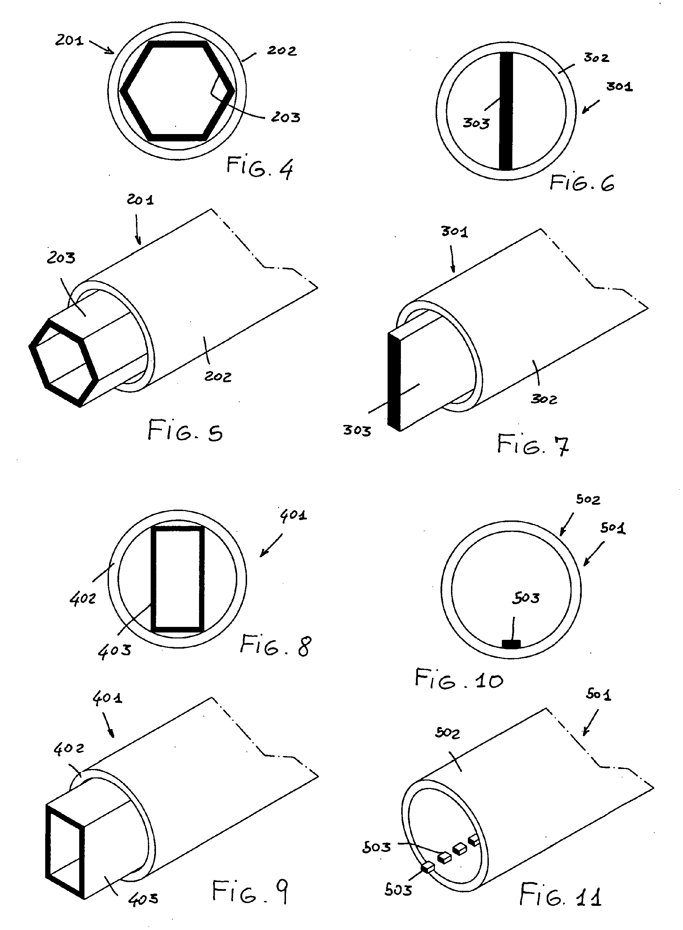

Figures 4 and 5 show a further bacteriostatically operating tube construction, according to the present invention, generally indicated by the reference number 201, comprising an extruded plastic material tubular body, generally indicated by the reference number 202, holding in its inside a silver element constituted by a polygonal cross-section tubular element 203.

Figures 6 and 7 show a further bacteriostatically operating tube according to the present invention, generally indicated by the reference number 301, comprising an extruded plastic material tubular body, generally indicated by the reference number 302, in which a silver element comprising a silver small bar or rod 303 is arranged.

Figures 8 and 9 show yet another bacteriostatically operating tube construction, according to the present invention, generally indicated by the reference number 401, comprising an extruded plastic material tubular body, generally indicated by the reference number 402, holding therein a silver element constituted by a rectangular cross-section tubular element 403.

Figures 10 and 11 show yet another bacteriostatically operating tube construction, according to the present invention, and being generally indicated by the reference number 501, comprising an extruded plastic material tubular body, generally indicated by the reference number 502, holding in its inside a silver element constituted by a plurality of silver pieces 503 which are linearly arranged along the inner wall of the tube.

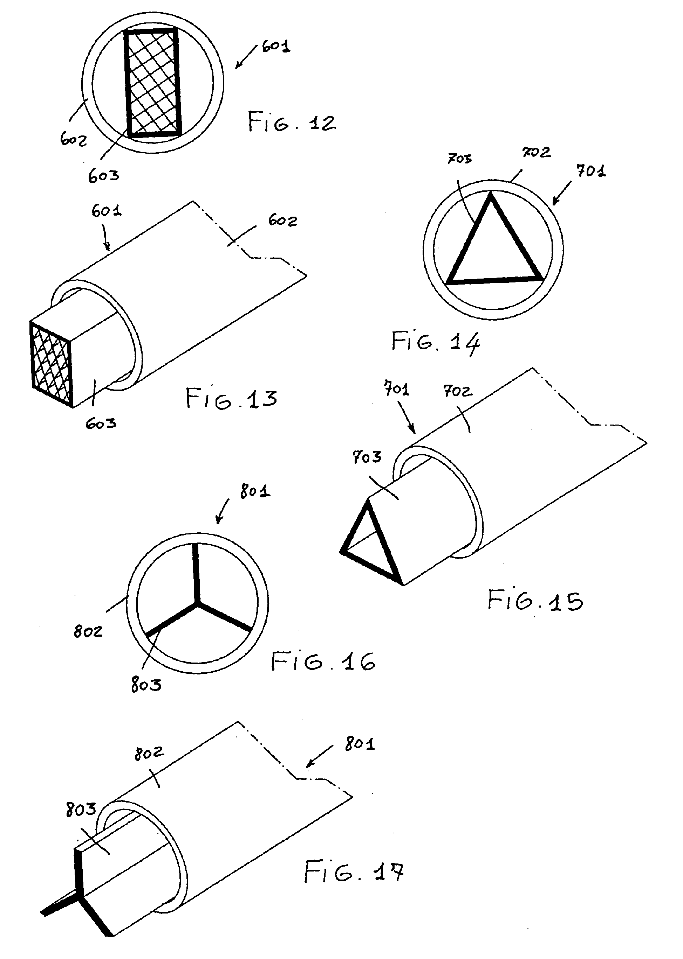

Figures 12 and 13 show yet another bacteriostatically operated tube construction, according to the present invention, and being generally indicated by the reference number 601, comprising an extruded plastic material tubular body, generally indicated by the reference number 602, holding in its inside a silver element 603, having a cell arrangement.

Figures 14 and 15 show yet another bracteriostatically operating tube construction, according to the present invention, and being generally indicated by the reference number 701, comprising an extruded plastic material tubular body, generally indicated by the reference number 702, holding in its inside a triangular cross-sectional tubular element 703, also made of silver.

Figures 16 and 17 show yet another bacteriostatically operating tube construction, according to the present invention, generally indicated by the reference number 801, comprising an extruded plastic material tubular body, generally indicated by the reference number 802, with an inner silver element including three longitudinal angled portions 803.

The herein shown and disclosed embodiments represent some possible structural examples of the bacteriostatically operating tube construction according to the present invention.

In this connection it should be apparent that the configuration or shape, arrangement and number of the silver elements inside the tube construction can be changed depending on requirements, without departing from the scope of the invention.

In practicing the invention, the used materials, as well as the contingent size and shapes, can be any, depending on requirements.

## Claims

1. A bacteriostatically operating tube construction, for food or medical use, **characterized in that** said tube construction comprises an extruded plastic material tubular body holding one or more inner silver elements.

2. A bacteriostatically operating tube construction, according to claim 1, **characterized in that** said tubular body is an extruded plastic material tubular body, which is extruded starting from a plastic material such as polyurethane, polyethylene, rilsan®, polyamide, hytrel, the tube construction being extruded and coated and, during the extrusion process, in said tubular body being formed and arranged one or more coil or spiral elements made of a 999/1000 silver wire.

3. A bacteriostatically operating tube construction, according to claim 1 or 2, **characterized in that** said silver wire has a silver rate of 999/1000, and a circular cross-section having a diameter of 0.5 mm.

4. A bacteriostatically operating tube construction, according to one or more of the preceding claims, **characterized in that** the pitch, i.e. the distance between two turns of the coil element, is of 20 mm for a tube construction having a diameter less than 12 mm.

5. A bacteriostatically operating tube construction, according to one or more of the preceding claims, **characterized in that** said tube construction has a load loss therethrough which is distributed with an increase of the load loss value of substantially 15 to 18% larger than that of the same tube construction without the inner spiral or coil element.

6. A bacteriostatically operating tube construction, according to claim 1, **characterized in that** said silver element comprise a silver wire linearly arranged inside said tube construction.

7. A bacteriostatically operating tube construction, according to claim 1, **characterized in that** said silver element comprises a tubular element having a polygonal cross-section.

8. A bacteriostatically operating tube construction, according to claim 1, **characterized in that** said silver element comprises a silver bar or rod.

9. A bacteriostatically operating tube construction, according to claim 1, **characterized in that** said silver element comprises a rectangular cross-section tubular element.

10. A bacteriostatically operating tube construction, according to claim 1, **characterized in that** said silver element comprises a plurality of silver pieces, said silver pieces being linearly arranged along an inner wall of said tube construction.

11. A bacteriostatically operating tube construction, according to claim 1, **characterized in that** said silver element comprises a cell element.

12. A bacteriostatically operating tube construction, according to claim 1, **characterized in that** said silver element comprises a tubular silver element having a triangular cross-section.

13. A bacteriostatically operating tube construction, according to claim 1, **characterized in that** said silver element comprises three longitudinal angled portions.

14. An apparatus for making a bacteriostatically operating tube construction, specifically designed for food or medical elements, comprising an extruded plastic material tubular body containing one or more inner elements and being **characterized in that** said apparatus comprises an extruding head having a central core allowing the silver element to be passed through and arranged inside the extruded plastic material tube construction.

15. An apparatus according to claim 14, **characterized in that** said passage is performed through a scroll path in order to facilitate the forming of the spiral silver coil and for evenly introducing said silver element into said tube construction.

16. An apparatus according to claim 15, **characterized in that** said apparatus comprises a timed feeding system providing a constant feeding of said silver wire or element depending on the plastic tube construction extruding speed.

17. An apparatus according to the preceding claim, **characterized in that** in said apparatus said coil or spiral element is locked inside said tube construction due to a combined action determined by a normal plastic material shrinking of said tube during the cooling of said tube, a very slight embedding of said silver element or wire in said plastic material as the latter is caused to solidify, and due to a very small increase of the silver spiral or coil element in a freed condition thereof.
